# EUROPEAN PATENT SPECIFICATION

(11) **EP 2 474 529 B1**
(45) Date of publication and mention of the grant of the patent: **14.05.2014**
(21) Application number: 10196836.0
(22) Date of filing: 23.12.2010
(51) Int. Cl.: C07D 209/08, A61K 31/404, A61P 13/02

(54) **Crystalline forms of an active pharmaceutical ingredient**
Kristallinformen von aktiven pharmazeutischen Inhaltsstoffen
Formes cristallines d'un ingrédient pharmaceutique actif

(43) Date of publication of application: 11.07.2012
(73) Proprietor: Sandoz AG, 4056 Basel (CH)
(72) Inventor: Pichler, Arthur, 6250 Kundl (AT); Hotter, Andreas, 6250 Kundl, (AT); Häfele, Clemens, 6020, Innsbruck (AT)
(74) Representative: Prüfer & Partner GbR European Patent Attorneys

(56) References cited:
- EP-A1- 1 541 554

## Description

### FIELD OF THE INVENTION

The present invention relates to the novel crystalline forms δ and ε of Silodosin, named form δ and form ε, and to methods for their preparation. Furthermore the present invention relates to the use of form δ for the preparation of form β and form ε. In addition the present invention relates to pharmaceutical compositions comprising an effective amount of crystalline forms δ and/or ε of Silodosin.

### BACKGROUND OF THE INVENTION

2,3-Dihydro-1-(3-hydroxypropyl)-5-[(2*R*)-2-[[2-[2-(2,2,2-trifluoroethoxy)phenoxy]ethyl]amino] propyl]-1*H*-indole-7-carboxamide, hereinafter designated as Silodosin, acts as an α₁-adrenoceptor antagonist and is useful as a therapeutic agent for dysuria (EP06006675A). Silodosin was launched in Japan (2006) for the treatment of dysuria associated with benign prostatic hypertrophy (BPH) under the brandname Urief^{®} and in the US (2009) for the treatment of the signs and symptoms of BPH under the brandname Rapaflo^{®}. In February 2010, the European Commission approved Silodosin (Silodyx^{®}) for the treatment of the signs and symptoms of BPH. Silodosin is administered orally and the chemical structure of Silodosin is displayed:

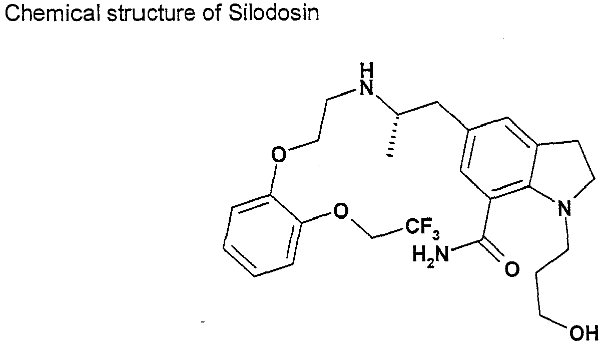

EP1541554A describes crystalline forms α, β and γ of Silodosin as well as the preparation thereof. The document provides stability and hygroscopicity data for all three polymorphs but is silent about solubility properties.

Polymorphism is a phenomenon relating to the occurrence of different crystal forms for one molecule. Different polymorphs may possess distinct advantageous in physical properties such as solubility, chemical stability, hygroscopicity, melting point, density, etc. According to the biopharmaceutical classification system the bioavailability of an oral drug substance depends on the permeability and the solubility of the drug substance. Thus polymorphic forms of Silodosin with high solubility and consequently high bioavailability are desired. It is however unforeseeable whether a further crystalline form exists, and if so how it can be obtained and which properties it has.

EP1541554A mentions that crystalline forms α, β and γ are suitable polymorphs for the preparation of oral solid medicaments due to their hygroscopicity and stability properties but form α is preferred because of certain issues with forms β and γ. For example, according to EP1541554A, form β has a manufacturing issue in industrial preparation when prepared according to the processes described in said document, in particular the preparation of form β requires a vigorous mixing of solvents in combination with a forcible and sudden precipitation. Hence, alternative processes for the preparation of crystalline form β which are industrially applicable are desired.

### SUMMARY OF THE INVENTION

The present invention relates to the novel crystalline forms of Silodosin, called form δ and form ε, and to methods for their preparation. Furthermore the present invention relates to the use of form δ for the preparation of form β and form ε. In addition the present invention relates to pharmaceutical compositions comprising an effective amount of crystalline form δ and/or form ε of Silodosin.

The present invention also provides an industrial applicable process for the preparation of form β via the novel form δ. Form δ transforms to form β upon heating and is thus a highly valuable form for the preparation of pure form β.

In addition form δ is an anhydrous non-hygroscopic and solvent free form of Silodosin and thus especially suitable for the preparation of pharmaceuticals.

Crystalline form ε shows higher solubility and thus higher bioavailability than crystalline forms α, β and γ of EP1541554. Crystalline form ε therefore is a highly valuable crystalline form for the preparation of pharmaceuticals.

Crystalline form ε is prepared from crystalline form δ. Hence, crystalline form δ is a highly valuable polymorph for the preparation of crystalline form ε.

### BRIEF DESCRIPTION OF THE DRAWINGS

Figure 1: X-ray powder diffraction (XRPD) pattern of crystalline form δ of Silodosin
Figure 2: Thermogravimetric analysis (TGA) curve of crystalline form δ of Silodosin
Figure 3: Differential scanning calorimetric (DSC) curve of crystalline form δ of Silodosin
Figure 4: Moisture sorption isotherms of crystalline form δ of Silodosin
Figure 5: X-ray powder diffraction (XRPD) pattern of crystalline form ε of Silodosin

### DETAILED DESCRIPTION OF THE INVENTION

In a first aspect, the present invention relates to a crystalline form of Silodosin (hereinafter also referred to as form δ).

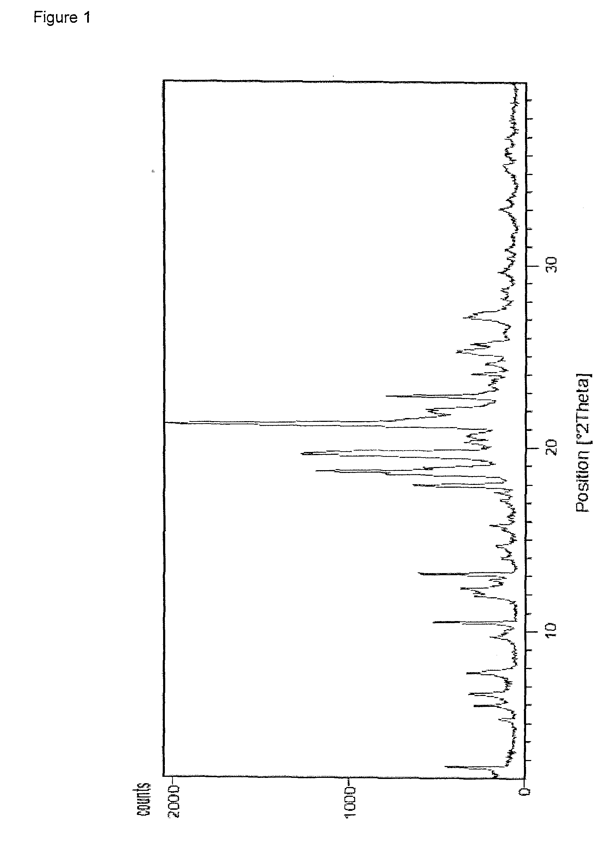

Form δ of Silodosin can be defined by an XRPD pattern comprising characteristic peaks at 2-theta angles of 6.6 ± 0.2°, 10.5 ± 0.2°, 13.1 ± 0.2°, 21.3 ± 0.2° and 22.8 ± 0.2°. Preferably, further peaks may be found, including peaks shown at 2-theta angles of 2.6 ± 0.2°, 6.6 ± 0.2°, 10.5 ± 0.2°, 13.1 ± 0.2°, 17.9 ± 0.2°, 18.7 ± 0.2°, 21.3 ± 0.2° and 22.8 ± 0.2°. An illustrative XRPD pattern of Silodosin form δ is displayed in figure 1.

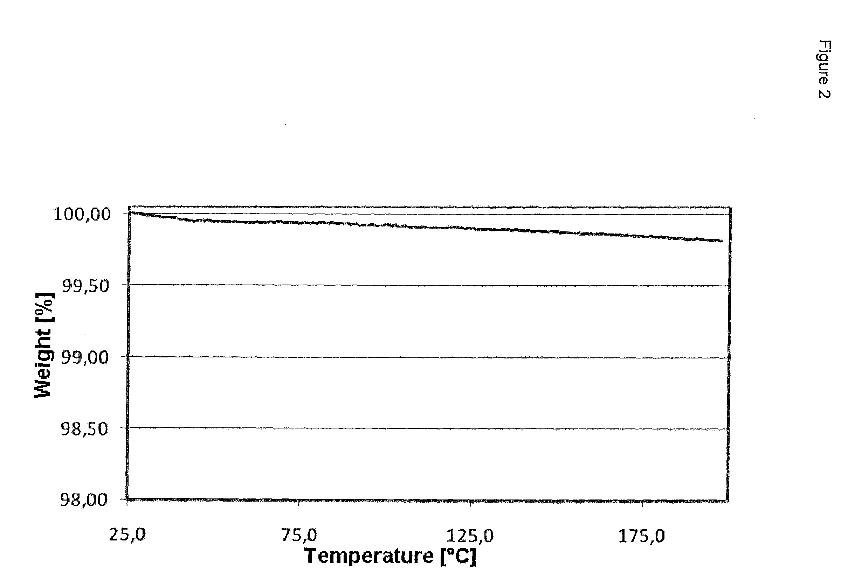

Silodosin form δ may optionally be further described by its thermogravimetric analysis. An illustrative TGA curve is displayed in figure 2. As displayed in figure 2 no significant mass loss (about 0.1 weight %) is observed up to the melting point at about 106°C, confirming the presence of a non-solvated anhydrous crystalline form of Silodosin.

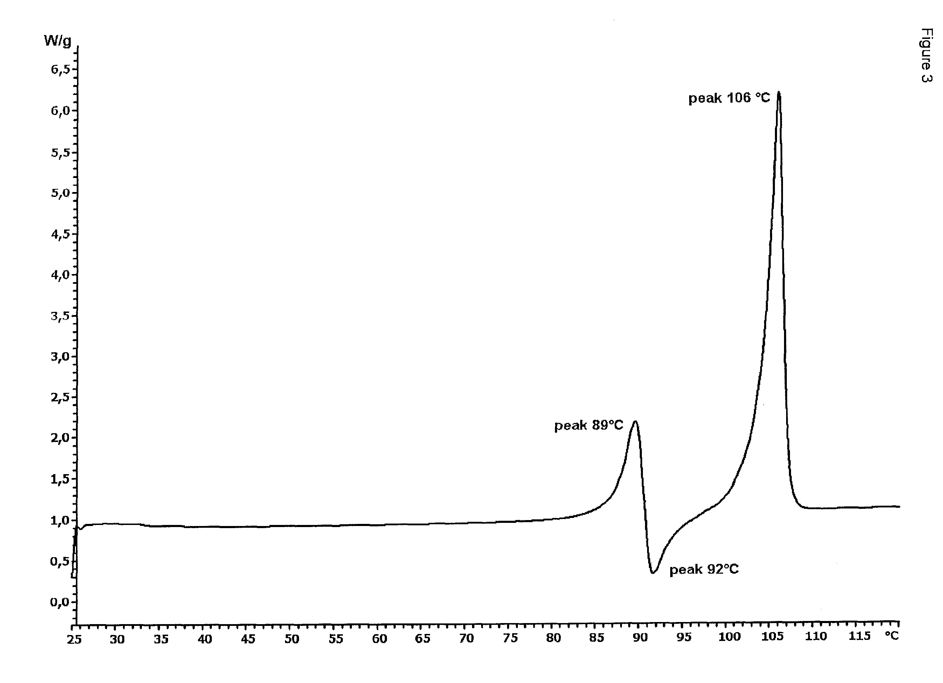

Form δ of Silodosin may optionally be further described by differential scanning calorimetric analysis. An illustrative DSC curve is displayed in figure 3. The DSC curve shows a first endotherm with a peak maximum at about 89°C immediately followed by an exotherm with a peak maximum at about 92 °C, which has been found to be due to a transformation of form δ to form β. The second endotherm with a peak maximum at about 106 °C is due to the melting process of form β.

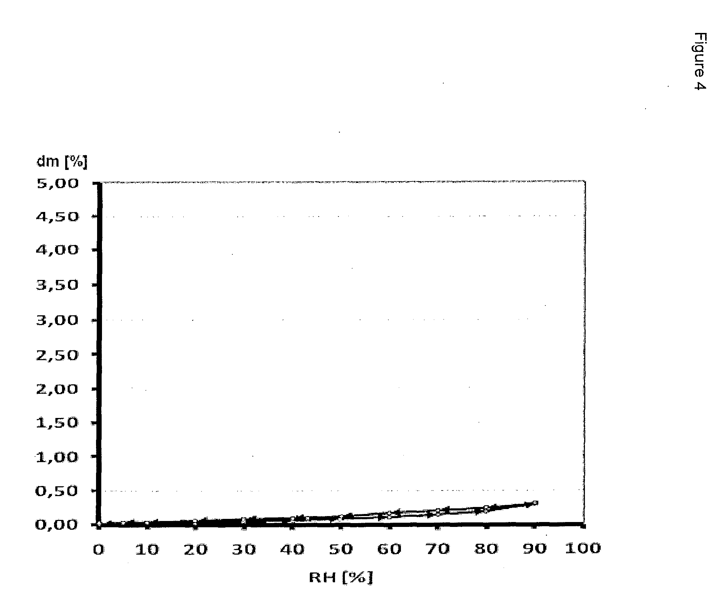

Further alternatively, Silodosin form δ may optionally be characterized by its moisture sorption isotherm, which is illustratively displayed in figure 4. Significantly, form δ of Silodosin can be classified as non-hygroscopic as it shows only a minimal reversible moisture uptake of about 0.35 weight % at about 90 % relative humidity.

The present invention also relates to a process for the preparation of form δ. Form δ of Silodosin may be prepared by a process comprising the steps of:
a) dissolving Silodosin in tetrahydrofuran,
b) optionally filtering the solution,
c) adding an anti-solvent, selected from the group consisting of n-heptane, n-hexane, cyclohexane and tert.-butylmethylether, to the solution,
d) stirring the obtained suspension,
e) optionally cooling the suspension,
f) isolating crystalline form δ and
g) drying crystalline form δ;

Any form of Silodosin may be applied in the dissolution step, e.g. amorphous Silodosin, crystalline Silodosin or a mixture of amorphous and crystalline Silodosin. Suitable crystalline forms of Silodosin are e.g. forms α, β and γ of EP1541554, form ε of the present invention or mixtures thereof.

Any form of Silodosin is easily solubilized in THF. Suitable initial concentrations range from about 10 to 200 g/L. The obtained solution may optionally be filtered in order to remove any undissolved particles.

The crystallization of form δ is initiated by adding n-heptane to the solution, as Silodosin is insoluble in n-heptane. While n-heptane is preferred, other suitable anti-solvents are e.g. n-hexane, cyclohexane or tert.-butylmethylether. The anti-solvent is added at room temperature or below, preferably at room temperature and the THF/anti-solvent ratio ranges from about 1.0:0.5 to 1.0:4.0 (v:v).

The obtained suspension is stirred at room temperature or below, preferably at room temperature, for about 6 to 72 hours, more preferably for about 6 to 48 hours and most preferably for about 6 to 24 hours.

Then the suspension may optionally be cooled in order to force more material out of the solution before the crystals are isolated by any conventional method such as filtration, centrifugation or evaporation of the solvent mixture.

Thereafter the crystalline form δ is dried preferably under vacuum at a temperature preferably ranging from about 25-60 °C, more preferably from about 30-55 °C and most preferably the material is dried at about 40-50 °C for a time ranging preferably from about 1-72 hours, more preferably from about 6-48 hours and most preferably from about 12-24 hours.

Surprisingly form δ easily transforms to pure form β upon heating at a temperature of about 80-100 °C, more preferably the transformation appears at a temperature of about 85-95 °C and most preferably form δ is transforms to form β at about 90°C. After transformation the sample is cooled to room temperature and form β is collected.
Form β shall be defined as disclosed in EP1541554, e.g showing characteristic peaks at 2-theta angles of 7.0 ± 0.2°, 12.5 ± 0.2°, 18.5 ± 0.2°, 19.5 ± 0.2°, 20.7 ± 0.2°and 21.1 ± 0.2°. A further reference is illustratively shown in Fig. 2 of EP1541554.

Form δ is substantially free from residual solvents as can be seen from the TGA-curve presented in figure 3. Therefore, subjecting form δ to about 90°C results in pure form β essentially free from residual solvents.

Hence form δ is a valuable polymorph for the manufacture of form β of Silodosin in industrial scale.

In a second aspect, the present invention relates to another crystalline form of Silodosin (hereinafter also referred to as form ε).

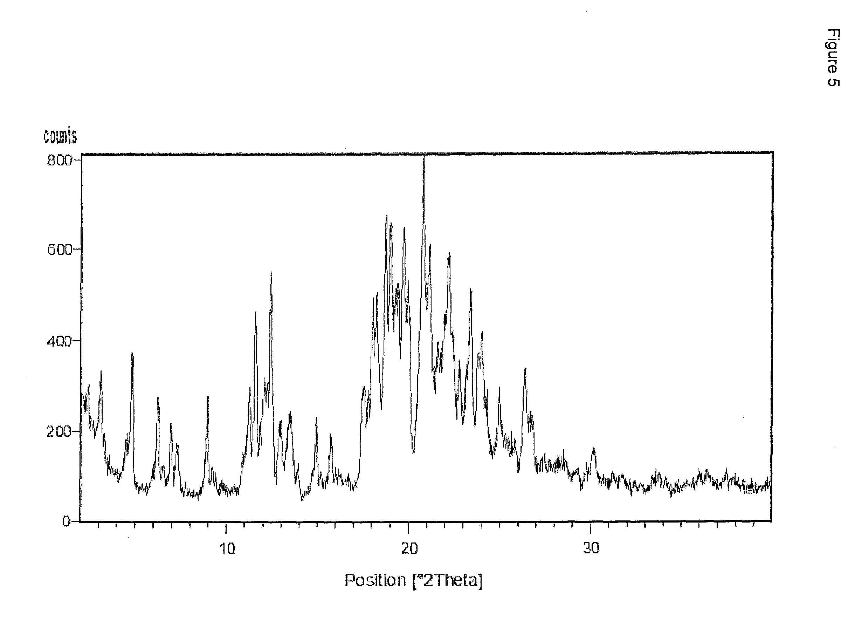

Form ε of Silodosin can be defined by an XRPD pattern comprising characteristic peaks at 2-theta angles of 3.1 ± 0.2°, 4.8 ± 0.2°, 6.2 ± 0.2°, 8.9 ± 0.2° and 11.6 ± 0.2°. Preferably, further peaks may be found, including peaks shown at 2-theta angles of 3.1 ± 0.2°, 4.8 ± 0.2°, 6.2 ± 0.2°, 8.9 ± 0.2°, 11.6 ± 0.2°, 18.2 ± 0.2°, 18.7 ± 0.2°, 19.7 ± 0.2°, 20.8 ± 0.2°, 22.2 ± 0.2°, 23.4 ± 0.2° and 26.4 ± 0.2°. An illustrative XRPD pattern of Silodosin form ε is displayed in figure 5.

The present invention also provides a process for the preparation of form ε. Form ε of Silodosin may be prepared from crystalline form δ described above by a process comprising the steps of:
a) slurrying form δ in aqueous methanol,
b) isolating form ε and
c) drying form ε;

Form δ is applied as starting material, as form ε is only obtained by slurrying form δ in aqueous methanol. Form ε could not be obtained by slurrying forms α, β or γ of EP1541554 in aqueous methanol.

The concentration of the aqueous methanol used for the process ranges from about 10-90 % (v:v), preferably from about 20-80 % (v:v), more preferably from about 30-70 % (v:v) and most preferably from about 40-60 % (v:v).

The transformation of crystalline form δ to form ε usually appears fast. For example after 0.5 hours a sample from the aqueous methanol suspension was withdrawn and identified as form ε by XRPD. Nevertheless, in order to have a robust process in place the suspension is preferably stirred for about 1-72 hours, more preferably for about 6-48 hours and most preferably for about 12-24 hours.

For the isolation step any conventional method such as filtration, centrifugation or evaporation of the solvent may be applied.

Crystalline form ε may be dried preferably under vacuum at a temperature preferably ranging from about 25-60 °C, more preferably from about 30-55 °C and most preferably the material is dried at about 40-50 °C for a time ranging preferably from about 1-72 hours, more preferably from about 6-48 hours and most preferably from about 12-24 hours.

Surprisingly crystalline form ε of the present invention shows higher solubility than the known polymorphs α, β and γ of EP1541554. Form ε showed the highest equilibrium solubility of the investigated crystalline forms in a methanol/water mixture. Equilibrium concentrations after 60 minutes in aqueous methanol are listed in table 1.

**Table 1: Equilibrium concentrations after 60 minutes**

| **Polymorph** | **Concentration** |
|---|---|
| α | 21.7 mg/ml |
| β | 23.2 mg/ml |
| γ | 22.2 mg/ml |
| ε | 25.5 mg/ml |

The solvent system methanol/water represents the dissolution of solid states of Silodosin in aqueous systems. All crystalline forms are kinetically stable in the test solution as they did not show any changes in their XRPD patterns after the dissolution experiment.

The crystalline forms δ and ε of Silodosin are polymorphically stable. For example forms δ and ε were stored at ambient temperature for 50 days and stressed at 40 °C for 7 days and did not undergo a solid-state phase transformation during this time.

The crystal forms δ and/or ε of Silodosin of the invention as described above may advantageously be employed in various pharmaceutical formulations for use in the treatment of dysuria and related diseases in accordance with the present invention. The present invention therefore also relates to a pharmaceutical composition which comprises the crystalline forms δ and/or ε of Silodosin as described above and a pharmaceutically acceptable carrier.

The present invention therefore also relates to a pharmaceutical composition comprising the crystalline form δ of Silodosin. Preferably, the present invention relates to such pharmaceutical compositions, wherein more than 95 % of Silodosin is stably present as form δ, more preferably wherein form δ is the only detectable crystalline form of Silodosin.

Furthermore the present invention relates to a pharmaceutical composition comprising the crystalline form ε of Silodosin. Preferably, the present invention relates to such pharmaceutical compositions, wherein more than 95 % of Silodosin is stably present as form ε, more preferably wherein form ε is the only detectable crystalline form of Silodosin.

"Stably present" as defined herein means that even after storage of the pharmaceutical composition for 180 days, and preferably even after storage for 2 years, the crystalline forms of Silodosin designated as forms δ or ε initially comprised in the pharmaceutical composition are still present as crystalline forms δ or ε after storage for the indicated period.

The pharmaceutical compositions of the invention comprising the crystalline forms δ and/or ε of Silodosin may further comprise one or more pharmaceutically acceptable excipients. Such excipients are preferably selected from the group consisting of fillers, sweeteners, buffering agents, glidants, flowing agents, flavouring agents, lubricants, preservatives, surfactants, wetting agents, binders, disintegrants and thickeners. Other excipients known in the field of pharmaceutical compositions may also be used. Furthermore, the pharmaceutical composition may comprise a combination of two or more excipients also within one of the members of the above mentioned group.
Examples of suitable excipients for pharmaceutical compositions of the invention comprising Silodosin forms δ and/or ε are given e.g. in EP1574215, which is herein incorporated by reference, in paragraphs [0027] to [0030].

Paragraph [0027] of EP1574215 discloses an example of the filler for the pharmaceutical compositions of the present invention comprising Silodosin forms δ and/or ε. The preferred filler, which can also be used for the pharmaceutical compositions of the present invention, is D-mannitol.

Paragraph [0028] of EP1574215 discloses examples of disintegrants for the pharmaceutical compositions of the present invention comprising Silodosin forms δ and/or ε. The preferred disintegrant, which can also be used for the pharmaceutical compositions of the present invention, is starch e.g. corn starch, low-substituted hydroxypropylcellulose and partially pregelatinized starch e.g PCS or starch 1500.

Paragraph [0030] of EP1574215 discloses examples of lubricants for the pharmaceutical compositions of the present invention comprising Silodosin forms δ and/or ε. The preferred lubricants, which can also be used for the pharmaceutical compositions of the present invention, are magnesium stearate, calcium stearate and talc.

If desired, a suitable surfactant, which can also be used for the pharmaceutical composition comprising forms δ and/or ε of the present invention, is sodium lauryl sulphate.

Examples of suitable processes for the preparation of the pharmaceutical compositions of the present invention are given e.g. in EP1574215, which is herein incorporated by reference, in paragraphs [0049] and [0050].

Concrete examples for the production of capsules or tablets of the present invention are given e.g. in EP1574215, paragraphs [0084] to [0086] or in EP1541554, paragraphs [0052] to [0063]. These examples can be repeated using forms δ and/or ε of Silodosin of the present invention.

Silodosin is known to be relatively unstable against light exposure. Thus capsules with light shielding materials are used or tablets are coated by a material with a light shielding effect, wherein titanium dioxide is preferably the light shielding material.

### EXAMPLES

The X-ray powder diffraction (XRPD) patterns were obtained with an X'Pert PRO diffractometer (PANalytical, Almelo, The Netherlands) equipped with a theta/theta coupled goniometer in transmission geometry, programmable XYZ stage with well plate holder, Cu-Kα_{1,2} radiation source (wavelength 0,15419 nm) with a focusing mirror, a 0.5° divergence slit, a 0.02° soller slit collimator and a 1° anti-scattering slit on the incident beam side, a 2 mm anti-scattering slit, a 0.02° soller slit collimator and a Nickel filter on the diffracted beam side and a solid state PIXcel detector. The diffractograms were recorded at a tube voltage of 40 kV, tube current of 40 mA, applying a stepsize of 0.013° 2-theta with 40 s per step in the angular range of 2° to 40° 2-theta. A typical precision of the 2-theta values is in the range of ± about 0.2° 2-theta. Thus a diffraction peak that appears at 5.0° 2-theta can appear between 4.8 and 5.2° 2-theta on most X-ray diffractometers under standard conditions.

Thermogravimetric analysis was performed on a Thermogravimetric-system TGA-7, Pyris-Software for Windows NT, (Perkin-Elmer, Norwalk, Ct., USA). 4.986 mg was weighed into a Platinum-sample holder (50 µL). Nitrogen was used as purge gas (sample purge: 20 mL/min, balance purge: 40 mL/min). Heating rate: 5 K/min.

Differential scanning calorimetry (DSC) was performed on a Mettler Toledo Polymer DSC. 1.69 mg sample was heated in 40 µL Al-pans with a pierced lid from 25 to 120 °C at a rate of 10 °C/min. Nitrogen (purge rate 10 mL/min) was used as purge gas.

The moisture sorption isotherms were recorded with a SPS-11 moisture sorption analyzer (MD Messtechnik, Ulm, D). The measurement cycle was started at 40 % relative humidity (RH), decreased in 10 % steps down to 0 % RH, increased in 10 % steps up to 90 % RH, decreased in 10 % steps down to 0 % RH and increased in 10 % steps to 40 % RH again. The equilibrium condition for each step was set to a mass constancy of ± 0.01 % over 30 min. The temperature was 25 ± 0.1 °C.

### Example 1: Preparation of Silodosin form δ

100 mg Silodosin were dissolved in 1 mL THF at room temperature. To the solution 1 mL n-heptane was added and the mixture was stirred at room temperature, whereas crystallization started within a few minutes. The suspension was stirred for additional 3 hours before the solid material was filtered off, washed with n-heptane and dried at 40 °C under vacuum for about 17 hours. According to the present process 70 mg (70 % yield) of crystalline form δ were obtained.

### Example 2: Preparation of Silodosin form δ

100 mg Silodosin were dissolved in 1 mL THF at room temperature. To the solution 2 mL n-heptane were added and the obtained suspension was stirred for 3 hours before the solid material was filtered off and dried at 40 °C under vacuum for about 20 hours. According to the present process 84 mg (84 % yield) of crystalline form δ were obtained.

### Example 3: Transformation of Silodosin form δ into form β

300 mg Silodosin form δ were weighed into a glass vial which was then sealed. The sample was stored at 90 °C for 1 hour. The material was allowed to cool to room temperature and XRPD confirmed the transformation to form β.

### Example 4: Preparation of Silodosin form ε from Silodosin form 8

1.060 g Silodosin form δ (obtained e.g. by the processes of examples 1 or 2) were suspended in 10 mL aqueous methanol 50 % (v:v) at room temperature. After 0.5 hours a sample was withdrawn from the suspension and identified as Silodosin form ε by XRPD. 5 mL MeOH 50 % (v:v) were added and the mixture was stirred for additional 68 hours. Finally the solid was filtered off and dried under vacuum at 40 °C to obtain crystalline form ε.

### Example 5: Solubility determination of Silodosin forms α, β, γ and ε

Approximately 400 mg of each crystalline form of Silodosin (α, β and γ were obtained according to the procedures described in EP1541554, ε was obtained according to example 3 herein) were stirred in 10 mL MeOH 50 % (v:v) at 25 ± 1 °C. Subsequently 1 mL was withdrawn from each suspension after 15, 30 and 60 min with the aid of a volumetric pipette and filtered. Filtrates were diluted to 50 mL with MeOH 50 % (v:v) and the respective concentrations were determined by UV-spectrophotometry at 269 nm (apparatus: Perkin Elmer Lambda 35).
A calibration curve was determined based on a series of known concentrations in the same solvent system.

### Result:

| **Polymorph** | **Concentration** |
|---|---|
| α | 21.7 mg/ml |
| β | 23.2 mg/ml |
| γ | 22.2 mg/ml |
| ε | 25.5 mg/ml |

## Claims

1. Crystalline form δ of Silodosin having an X-ray powder diffraction pattern comprising peaks at 2- theta angles of 6.6 ± 0.2°, 10.5 ± 0.2°, 13.1 ± 0.2°, 21.3 ± 0.2° and 22.8 ± 0.2°.

2. The crystalline form of Silodosin according to claim 1 **characterized by** a DSC curve showing an endotherm peak at 89 °C and an exotherm peak at 92 °C.

3. The crystalline form of Silodosin according to claim 1 **characterized by** a non-hygroscopic property indicated by a reversible moisture uptake of below 0.5 weight % from 0 % to 90 % relative humidity at 25 ±0.1 °C.

4. A method for the preparation of the crystalline form of Silodosin according to any one of claims 1 to 3 comprising the steps of:
a) dissolving Silodosin in tetrahydrofuran,
b) optionally filtering the solution,
c) adding an anti-solvent, selected from the group consisting of n-heptane, n-hexane, cyclohexane and tert.-butylmethylether, to the solution,
d) stirring the obtained suspension,
e) optionally cooling the obtained suspension,
f) isolating the crystalline form of Silodosin according to any one of claims 1 to 3 and
g) drying the crystalline form of Silodosin according to any one of claims 1 to 3.

5. Crystalline form ε of Silodosin having an X-ray powder diffraction pattern comprising peaks at 2- theta angles of 3.1 ± 0.2°, 4.8 ± 0.2°, 6.2 ± 0.2°, 8.9 ± 0.2° and 11.6 ± 0.2°.

6. A method for the preparation of the crystalline form of Silodosin according to claim 5 comprising the steps of:
a) slurrying the crystalline form of Silodosin according to any one of claims 1 to 3 in aqueous methanol,
b) isolating the crystalline form of Silodosin according to claim 5 and
c) drying the crystalline form of Silodosin according to claim 5.

7. A pharmaceutical composition comprising a crystalline form of Silodosin according to any one of claims 1 to 3, and/or comprising a crystalline form of Silodosin according to claim 5, further comprising at least one pharmaceutically acceptable excipient.

8. The pharmaceutical composition according to claim 7, which is an oral dosage form, in particular a capsule or tablet.

9. Use of a crystalline form of Silodosin according to any one of claims 1 to 3 or claim 5 for the production of a pharmaceutical composition.

10. The pharmaceutical composition as defined in any of the claims 7 and 8, or as obtained by the use according to claim 9, for use in the treatment of dysuria.

11. Use of the crystalline form of Silodosin as defined in any of the claims 1 to 3 as an intermediate in a process for the preparation of Silodosin form β.

12. A process for the preparation of crystalline form β of Silodosin, comprising heating the crystalline form of Silodosin according to any one of claims 1 to 3 to a temperature of 80 °C to 100 °C.

13. A process for preparing a pharmaceutical composition comprising crystalline form β of Silodosin, comprising the steps of carrying out a process according to claim 12, and mixing form β of Silodosin with at least one pharmaceutically acceptable excipient.

14. Use of the crystalline form of Silodosin as defined in any one of the claims 1 to 3 as an intermediate in a process for the production of a crystalline form of Silodosin according to claim 5.

## Patentansprüche

1. Kristallform δ von Silodosin mit einem Röntgenpulverbeugungsmuster umfassend Peaks bei 2-Theta-Winkeln von 6,6 + 0,2°, 10,5 ± 0,2°, 13,1 ± 0,2°, 21,3 ± 0,2° und 22,8 ± 0,2°.

2. Kristallform von Silodosin gemäß Anspruch 1, **gekennzeichnet durch** eine DSC-Kurve, welche einen endothermen Peak bei 89°C und einen exothermen Peak bei 92°C aufweist.

3. Kristallform von Silodosin gemäß Anspruch 1, **gekennzeichnet durch** eine nicht-hygroskopische Eigenschaft, angezeigt **durch** eine reversible Feuchtigkeitsaufnahme von unter 0,5 Gewichts-% von 0% bis 90% relativer Luftfeuchtigkeit bei 25 + 0,1°C.

4. Verfahren zur Herstellung der Kristallform von Silodosin gemäß einem der Ansprüche 1 bis 3, umfassend die Schritte:
a) Auflösen von Silodosin in Tetrahydrofuran,
b) wahlweise Filtrieren der Lösung,
c) Zugeben eines Anti-Lösungsmittels, ausgewählt aus der Gruppe bestehend aus n-Heptan, n-Hexan, Cyclohexan und tert.-Butylmethylether zu der Lösung,
d) Rühren der erhaltenen Suspension,
e) wahlweise Abkühlen der erhaltenen Suspension,
f) Isolieren der Kristallform von Silodosin gemäß einem der Ansprüche 1 bis 3 und
g) Trocknen der Kristallform von Silodosin gemäß einem der Ansprüche 1 bis 3.

5. Kristallform ε von Silodosin mit einem Röntgenpulverbeugungsmuster umfassend Peaks bei 2-Theta-Winkeln von 3,1 ± 0,2°, 4,8 ± 0,2°, 6,2 ± 0,2°, 8,9 ± 0,2° und 11,6 ± 0,2°.

6. Verfahren zur Herstellung der Kristallform von Silodosin gemäß Anspruch 5, umfassend die Schritte:
a) Aufschlämmen der Kristallform von Silodosin gemäß einem der Ansprüche 1 bis 3 in wässrigem Methanol,
b) Isolieren der Kristallform von Silodosin gemäß Anspruch 5 und
c) Trocknen der Kristallform von Silodosin gemäß Anspruch 5.

7. Pharmazeutische Zusammensetzung umfassend eine Kristallform von Silodosin gemäß einem der Ansprüche 1 bis 3 und/oder umfassend eine Kristallform von Silodosin gemäß Anspruch 5, des Weiteren umfassend mindestens einen pharmazeutisch verträglichen Hilfsstoff.

8. Pharmazeutische Zusammensetzung gemäß Anspruch 7, welche eine orale Dosierungsform, insbesondere eine Kapsel oder Tablette ist.

9. Verwendung einer Kristallform von Silodosin gemäß einem der Ansprüche 1 bis 3 oder Anspruch 5 zur Herstellung einer pharmazeutischen Zusammensetzung.

10. Pharmazeutische Zusammensetzung wie in einem der Ansprüche 7 oder 8 definiert, oder wie durch die Verwendung gemäß Anspruch 9 erhalten, zur Verwendung in einer Behandlung von Dysurie.

11. Verwendung einer Kristallform von Silodosin wie in einem der Ansprüche 1 bis 3 definiert, als Zwischenprodukt in einem Verfahren zur Herstellung von Silodosin Form β.

12. Verfahren zur Herstellung der Kristallform β von Silodosin, umfassend ein Erhitzen der Kristallform von Silodosin gemäß einem der Ansprüche 1 bis 3 auf eine Temperatur von 80°C bis 100°C.

13. Verfahren zur Herstellung einer pharmazeutischen Zusammensetzung aufweisend eine Kristallform β von Silodosin, umfassend die Schritte zum Durchführen eines Verfahrens nach Anspruch 12 und des Mischens der Form β von Silodosin mit mindestens einem pharmazeutisch verträglichen Hilfsstoff.

14. Verwendung der Kristallform von Silodosin wie in einem der Ansprüche 1 bis 3 definiert, als ein Zwischenprodukt in einem Verfahren zur Herstellung einer Kristallform von Silodosin gemäß Anspruch 5.

## Revendications

1. Forme cristalline δ de silodosine ayant un diagramme de diffraction des rayons X sur poudre comprenant des pics à des angles 2 theta de 6,6 ± 0,2°, 10,5 ± 0,2°, 13,1 ± 0,2°, 21,3 ± 0,2° et 22,8 ± 0,2°.

2. Forme cristalline de silodosine selon la revendication 1, **caractérisée par** une courbe DSC ayant un pic d'endotherme à 89°C et un pic d'exotherme à 92°C.

3. Forme cristalline de silodosine selon la revendication 1, **caractérisée par** une propriété non hygroscopique indiquée par une reprise d'humidité réversible de moins de 0,5 % en poids de 0 % à 90 % d'humidité relative à 25 ± 0,1 °C.

4. Procédé de préparation de la forme cristalline de silodosine selon l'une quelconque des revendications 1 à 3, comprenant les étapes de :
a) dissolution de silodosine dans du tétrahydrofurane,
b) filtration facultative de la solution,
c) addition d'un antisolvant, choisi dans le groupe consistant en n-heptane, n-hexane, cyclohexane et tert-butylméthyléther, à la solution,
d) agitation de la suspension obtenue,
e) refroidissement facultatif de la suspension obtenue,
f) isolement de la forme cristalline de silodosine selon l'une quelconque des revendications 1 à 3, et
g) séchage de la forme cristalline de silodosine selon l'une quelconque des revendications 1 à 3.

5. Forme cristalline ε de silodosine ayant un diagramme de diffraction des rayons X sur poudre comprenant des pics à des angles 2 theta de 3,1 ± 0,2°, 4,8 ± 0,2°, 6,2 ± 0,2°, 8,9 ± 0,2° et 11,6 ± 0,2°.

6. Procédé de préparation de la forme cristalline de silodosine selon la revendication 5, comprenant les étapes de :
a) conversion en boue de la forme cristalline de silodosine selon l'une quelconque des revendications 1 à 3 dans du méthanol aqueux,
b) isolement de la forme cristalline de silodosine selon la revendication 5, et
c) séchage de la forme cristalline de silodosine selon la revendication 5.

7. Composition pharmaceutique comprenant une forme cristalline de silodosine selon l'une quelconque des revendications 1 à 3 et/ou comprenant une forme cristalline de silodosine selon la revendication 5, comprenant en outre au moins un excipient pharmaceutiquement acceptable.

8. Composition pharmaceutique selon la revendication 7, qui est une forme posologique orale, en particulier une capsule ou un comprimé.

9. Utilisation d'une forme cristalline de silodosine selon l'une quelconque des revendications 1 à 3 ou la revendication 5, pour la production d'une composition pharmaceutique.

10. Composition pharmaceutique selon l'une quelconque des revendications 7 et 8, ou telle qu'obtenue par l'utilisation selon la revendication 9, pour une utilisation dans le traitement de la dysurie.

11. Utilisation de la forme cristalline de silodosine telle que définie dans l'une quelconque des revendications 1 à 3, comme intermédiaire dans un procédé de préparation de silodosine forme β.

12. Procédé de préparation de la forme cristalline β de silodosine, comprenant le chauffage de la forme cristalline de silodosine selon l'une quelconque des revendications 1 à 3 à une température de 80°C à 100°C.

13. Procédé de préparation d'une composition pharmaceutique comprenant la forme cristalline β de silodosine, comprenant les étapes de réalisation d'un procédé selon la revendication 12, et de mélange de la forme β de silodosine avec au moins un excipient pharmaceutiquement acceptable.

14. Utilisation de la forme cristalline de silodosine telle que définie dans l'une quelconque des revendications 1 à 3, comme intermédiaire dans un procédé de production d'une forme cristalline de silodosine selon la revendication 5.
